# EUROPÄISCHE PATENTANMELDUNG

(11) **EP 4 591 819 A1**
(43) Veröffentlichungstag der Anmeldung: **30.07.2025**
(21) Anmeldenummer: 24153618.4
(22) Anmeldetag: 24.01.2024
(51) Int. Cl.: A61B 34/20, A61B 90/10, A61B 90/00, A61B 17/00, A61B 90/50

(54) **BEFESTIGUNGSVORRICHTUNG FÜR EINE THERAPIEVORRICHTUNG**

(71) Anmelder: Medivation AG, 5400 Baden (CH)
(72) Erfinder: STIFTER, Jan, 5425 Schneisingen (CH); Schwägli, Tobias, 4500 Solothurn (CH)
(74) Vertreter: Herrmann, Johanna

(57) **Zusammenfassung**

Eine Befestigungsvorrichtung (2) zur Befestigung einer Therapievorrichtung in einer in einem Knochen angeordneten Öffnung (9) enthält ein Hülsenelement (10), welches zur Aufnahme in der Öffnung (9) ausgebildet ist, enthaltend ein erstes Hülsenteilelement (11) und ein zweites Hülsenteilelement (12). Das erste Ende (14) des ersten Hülsenteilelements (11) umfasst ein Griffelement (18) und das zweite Ende (15) einen Fortsatz (19). Das zweite Hülsenteilelement (12) enthält eine Vorsprunganordnung (25, 26). Das erste Hülsenteilelement (11) und das zweite Hülsenteilelement (12) sind derart umeinander drehbar, dass die Vorsprunganordnung (25, 26) in einer ersten Stellung angrenzend an den Fortsatz (19) angeordnet ist und in einer zweiten Stellung einen Abstand zum Fortsatz (19) aufweist, der mindestens 5 % des Umfangs entspricht.

## Beschreibung

### Hintergrund

Die vorliegende Erfindung betrifft eine Befestigungsvorrichtung für eine Therapievorrichtung, beispielsweise ein chirurgisches Hilfsmittel oder ein Navigationshilfsmittel, insbesondere einen Tracker für die chirurgische Navigation. Die Befestigungsvorrichtung ist dazu ausgebildet, an einem Knochen befestigt zu werden.

### Stand der Technik

Befestigungsvorrichtungen von Tracking-Geräten an Knochen für die chirurgische Navigation werden in vielen bestehenden Anwendungen benötigt und eingesetzt.

Die Befestigung von Trackern für die chirurgische Navigation mittels einer Schädelklemme wird für mehrere neurologische Eingriffe beschrieben. Im Dokument US10433763B2 wird beispielsweise ein System beschrieben, bei dem ein Tracker an einer Schädelklemme befestigt wird. Andere Systeme verwenden eine Schädelfixierung mit Schrauben und Stiften, die Tracker am Schädelknochen befestigen (z. B. Brainlab Instrument "Reference Base Skull"). Die vorbekannten Befestigungsvorrichtungen ermöglichen eine starre Befestigung von Trackern am Knochen ausserhalb des Bereichs des chirurgischen Eingriffs und erfordern zusätzliche Schnitte und Bohrungen oder die Platzierung von Schrauben und/oder Klammern.

Bei den meisten kranialen Eingriffen wird der Zugang zum Gehirn durch das Bohren einer oder mehrerer Öffnungen, auch Bohrlöcher genannt, geschaffen. Diese Öffnungen haben normalerweise einen Durchmesser im Bereich von 5 bis einschliesslich 20 mm und ermöglichen den Zugang zum Gehirn durch ein Portal. Wenn grössere Teile des Schädels entfernt werden müssen, werden mehrere Öffnungen vorgesehen, die dann mit einer Säge verbunden werden. Lösungen für die Befestigung von Instrumenten oder Zielgeräten an solchen Öffnungen wird beispielsweise in den Dokumenten US9675783B2, US10307218B2 oder US10905497B2 beschrieben.

### Aufgabe der Erfindung

Aufgabe der Erfindung ist es, eine Befestigungsvorrichtung bereit zu stellen, mittels welcher eine Therapievorrichtung in einer Öffnung im Schädelknochen eines Patienten befestigt werden kann, wobei die Befestigungsvorrichtung ermöglicht, eine Therapievorrichtung nachträglich anzubringen oder zu entfernen, ohne die Befestigungsvorrichtung zu entfernen.

### Beschreibung der Erfindung

Die Lösung der Aufgabe der Erfindung erfolgt durch eine Befestigungsvorrichtung gemäss Anspruch 1. Vorteilhafte Varianten der Befestigungsvorrichtung sind Gegenstand der Ansprüche 2 bis 15.

Wenn der Begriff "beispielsweise" in der nachfolgenden Beschreibung verwendet wird, bezieht sich dieser Begriff auf Ausführungsbeispiele und/oder Ausführungsformen, was nicht notwendigerweise als eine bevorzugtere Anwendung der Lehre der Erfindung zu verstehen ist. In ähnlicher Weise sind die Begriffe "vorzugsweise", "bevorzugt" zu verstehen, indem sie sich auf ein Beispiel aus einer Menge von Ausführungsbeispielen und/oder Ausführungsformen beziehen, was nicht notwendigerweise als eine bevorzugte Anwendung der Lehre der Erfindung zu verstehen ist. Dementsprechend können sich die Begriffe "beispielsweise", "vorzugsweise" oder "bevorzugt" auf eine Mehrzahl von Ausführungsbeispielen und/oder Ausführungsformen beziehen.

Die nachfolgende detaillierte Beschreibung enthält verschiedene Ausführungsbeispiele für die erfindungsgemässe Befestigungsvorrichtung. Die Beschreibung einer bestimmten Befestigungsvorrichtung oder eines bestimmten Sicherheitssystems oder eines bestimmten Abweisers ist nur als beispielhaft anzusehen. In der Beschreibung und den Ansprüchen werden die Begriffe "enthalten", "umfassen", "aufweisen" als "enthalten, aber nicht beschränkt auf' interpretiert.

Eine erfindungsgemässe Befestigungsvorrichtung zur Befestigung einer Therapievorrichtung in einer in einem Knochen angeordneten Öffnung enthält ein Hülsenelement, welches zur Aufnahme in der Öffnung ausgebildet ist. Das Hülsenelement enthält ein erstes Hülsenteilelement und ein zweites Hülsenteilelement. Das erste Hülsenteilelement umgibt im Einbauzustand das zweite Hülsenteilelement. Das erste Hülsenteilelement ist als ein zylinderförmiger Hohlkörper mit einer Längsachse ausgebildet. Das erste Ende des ersten Hülsenteilelements umfasst ein Griffelement. Das zweite Ende des ersten Hülsenteilelements umfasst einen Fortsatz. Das zweite Hülsenteilelement ist als ein zylinderförmiger Hohlkörper mit einer Längsachse ausgebildet. Das zweite Hülsenteilelement enthält eine Vorsprunganordnung. Das erste Hülsenteilelement und das zweite Hülsenteilelement sind derart umeinander drehbar, dass die Vorsprunganordnung in einer ersten Stellung angrenzend an den Fortsatz angeordnet ist und die Vorsprunganordnung in einer zweiten Stellung einen Abstand zum Fortsatz aufweist.

Insbesondere kann der Abstand mindestens 5 % des Umfangs entsprechen. Je grösser der Abstand zwischen der Vorsprunganordnung und dem Fortsatz ausgebildet ist, desto grösser ist der Platzbedarf der Befestigungsvorrichtung in der Öffnung. Die Befestigungsvorrichtung ist somit für eine Öffnung verwendbar, deren Durchmesser eine Abweichung zum Nenndurchmesser aufweist. Diese Abweichung kann insbesondere in einem Bereich von bis zu einem Millimeter liegen, wenn der Durchmesser der Öffnung im Bereich von 10 mm bis einschliesslich 15 mm liegt. Je nach dem tatsächlich vorliegenden Durchmesser der Öffnung kann der Abstand zwischen der Vorsprunganordnung und dem Absatz entsprechend angepasst werden. Die Befestigungsvorrichtung ist somit einstellbar, sodass Ungenauigkeiten bei der Herstellung der Öffnung durch die Befestigungsvorrichtung ausgeglichen werden können. Mit anderen Worten kann der Drehwinkel, um welchen das Griffelement mit einem mit dem zweiten Hülsenteilelement verbundenen Halteelement einschliesst, einstellbar sein. Das Griffelement kann relativ zu dem Halteelement insbesondere bis zu einem Anschlag des Halteelements gedreht werden. Der Anschlag des Halteelements ist insbesondere derart angeordnet, dass der Fortsatz und die Vorsprunganordnung den maximal möglichen Abstand zueinander einnehmen. Insbesondere kann der maximal mögliche Abstand fast 50% des Umfangs entsprechen. Insbesondere kann die Vorsprunganordnung in der zweiten Stellung gegenüberliegend zum Fortsatz angeordnet sein.

Die Erfindung bezieht sich somit auf eine Befestigungsvorrichtung zur Befestigung einer Therapievorrichtung, beispielsweise eines chirurgischen Hilfsmittels, insbesondere eines Instruments, beispielsweise eines chirurgischen Trackers, welche dazu ausgebildet ist, an einem Knochen, beispielsweise einem Schädelknochen befestigt zu werden. Die Befestigungsvorrichtung ist speziell dafür ausgelegt, in einer Öffnung, in welcher beispielsweise ein chirurgischer Eingriff vorgenommen werden kann, beispielsweise einem Bohrloch im Schädelknochen, befestigt zu werden. Die Befestigungsvorrichtung enthält eine Öffnung, sodass auch eine chirurgische Navigation der Therapievorrichtung direkt durch die Befestigungsvorrichtung hindurch ermöglicht ist. Die in der Öffnung des Knochens angeordnete Befestigungsvorrichtung erfordert keinen zusätzlichen Einschnitt oder andere Arten der Fixierung, wie Schrauben oder Klammern.

Gemäss eines Ausführungsbeispiels enthält das erste Hülsenteilelement einen ersten Flansch sowie einen zweiten Flansch. Mittels des ersten und zweiten Flansches ist eine zusätzliche Fixierung und Stabilisierung der Befestigungsvorrichtung ermöglicht. Eine solche Fixierung kann auf verschiedene Weise realisiert werden, wobei insbesondere der zweite Flansch teilweise oder vollständig auf der Öffnung aufliegen kann. Insbesondere kann der zweite Flansch die Funktion eines Kragens aufweisen. Der Kragen verhindert, dass die Befestigungsvorrichtung zu weit in die Öffnung eingeführt wird und darunter liegendes Gewebe beschädigen kann. Zudem kann der Kragen zusätzliche Stabilität bieten. Mittels des Kragens ist eine definierte Positionierung der Befestigungsvorrichtung in der Einbauposition in der Öffnung ermöglicht. Insbesondere ein Schädelknochen kann einen härteren Knochenteil mit einer Dicke von wenigen Millimetern aufweisen. Der zweite Flansch kann gemäss eines nicht dargestellten Ausführungsbeispiels über mindestens eine Lasche mit einer Öffnung zur Aufnahme eines Befestigungsmittels, beispielsweise einer Schraube, aufweisen. Mittels der Schraube kann eine verbesserte und dauerhafte Fixierung der Befestigungsvorrichtung gewährleistet werden.

Der erste Flansch und der zweite Flansch sind gemäss eines Ausführungsbeispiels in einem Abstand zueinander angeordnet. Der Abstand zwischen dem ersten und dem zweiten Flansch entspricht der Dicke einer den Knochen bedeckenden Gewebeschicht. Insbesondere kann der Abstand zwischen dem ersten Flansch und dem zweiten Flansch mindestens 5 mm betragen. Der erste Flansch kann auf der Gewebeschicht aufliegen. Insbesondere kann auch das Griffelement auf der Gewebeschicht aufliegen, sodass die Betätigung des Griffelements keine Verletzung der Gewebeschicht zur Folge haben kann.

Eine zusätzliche Profilierung an den Kontaktflächen insbesondere der Vorsprunganordnung und/oder des Fortsatzes kann eine stabilere Fixierung der Befestigungsvorrichtung am Knochen ermöglichen. Mit Kontaktflächen sind die Oberflächen der Befestigungsvorrichtung gemeint, die im Einbauzustand im Kontakt mit dem Knochen stehen. Die Profilierung kann mindestens ein Element aus der Gruppe bestehend aus Rippen, Rillen, Zähnen, Spikes, Spitzen, Schneidelementen oder Gewinden umfassen.

Gemäss eines Ausführungsbeispiels erfolgt eine Verwendung eines Press-Fit-Designs, bei dem der Querschnitt des Hülsenelements zumindest an den Kontaktpunkten oder Kontaktflächen der Innenwand der Öffnung etwas grösser als der Querschnitt der Öffnung ist, sodass das Hülsenelement zumindest an den Kontaktpunkten oder Kontaktflächen gegen die Innenwand gepresst wird. Ein Press-fit-Design kann auch mittels eines zusätzlichen Montagehilfsmittels bzw. Demontagehilfsmittels wie beispielsweise eines Inserters oder eines Retraktors, realisiert werden. Das Montageihilfsmittel kommt zum Einsatz, wenn die Befestigungsvorrichtung in der Öffnung eingesetzt wird. Das Demontagehilfsmittel kommt beim Entfernen der Befestigungsvorrichtung zum Einsatz.

Gemäss eines Ausführungsbeispiels überragt der Fortsatz den zweiten Flansch in Richtung der Längsachse des zylinderförmigen Hohlkörpers des ersten Hülsenteilelements.

Gemäss eines Ausführungsbeispiels erstreckt sich der Fortsatz über einen Teil eines Umfangs des ersten Hülsenteilelements. Eine Befestigungsvorrichtung mit einem Fortsatz, der sich nur über einen kleinen Teil des Umfangs des Hülsenelements erstreckt, ist eine besonders einfache Einführung des Hülsenelements in die Öffnung ermöglicht.

Gemäss eines Ausführungsbeispiels weist der Fortsatz eine Segmentlänge auf, die im Bereich von 10% bis einschliesslich 40% des Umfangs des zylinderförmigen Hohlkörpers liegt. Die Segmentlänge des Fortsatzes ermöglicht es, dass die Befestigungsvorrichtung an den Durchmesser der Öffnung anpassbar ist. Der Durchmesser der Öffnung im Knochen kann nicht exakt auf das entsprechende Nennmass hergestellt sein, da der Knochen nicht homogen und als organisches Material nicht immer von derselben Konsistenz ist. Daher unterliegt der effektive Durchmesser einer Abweichung, die im Bereich von ungefähr 1 mm liegt, wenn der Nenndurchmesser im Bereich von 10 bis einschliesslich 15 mm liegt. Mittels der erfindungsgemässen Befestigungsvorrichtung ist somit ein Toleranzausgleich ermöglicht. Je nach dem effektiven Durchmesser der Öffnung kann der Fortsatz gerade so weit von der Vorsprunganordnung weg bewegt werden, bis ein merklicher Widerstand spürbar ist. Wenn das Griffelement nicht mehr ohne erheblichen Kraftaufwand weiter gedreht werden kann, ist die Befestigungsvorrichtung in der Öffnung fixiert.

Gemäss eines Ausführungsbeispiels weist der Fortsatz eine axiale Länge auf, die im Bereich von 5% bis einschliesslich 50% des zwischen dem ersten Ende und einem dem Ende in Richtung einer Längsachse des ersten Hülsenteilelements gemessenen Abstandes liegt.

Gemäss eines Ausführungsbeispiels weist der Fortsatz eine Profilierung auf der Aussenseite auf. Die Profilierung kann ein Eingreifen des Fortsatzes in der Innenwand der Öffnung ermöglichen, wodurch eine verbesserte Fixierung der Befestigungsvorrichtung in der Öffnung erzielbar ist.

Gemäss eines Ausführungsbeispiels umfasst die Vorsprunganordnung einen ersten Vorsprung und einen zweiten Vorsprung. Jeder der ersten und zweiten Vorsprünge weist insbesondere eine Segmentlänge auf, die im Bereich von 1% bis maximal 10% des Umfangs beträgt. Der Abstand zwischen dem ersten Vorsprung und dem zweiten Vorsprung entspricht mindestens der Segmentlänge eines der ersten oder zweiten Vorsprünge.

Gemäss eines Ausführungsbeispiels enthält die Vorsprunganordnung eine Profilierung. Insbesondere weist die äussere Oberfläche der Vorsprunganordnung die Profilierung auf. Die Profilierung kann mindestens ein Element aus der Gruppe bestehend aus Rippen, Rillen, Zähnen, Spikes, Spitzen, Schneidelementen oder Gewinden umfassen.

Gemäss eines Ausführungsbeispiels weist das zweite Hülsenteilelement eine Aussenseite auf, die zumindest einen Absatz enthält. Mittels des Absatzes ist eine genaue Positionierung des Halteelements auf dem zweiten Hülsenteilelement ermöglicht. Das Halteelement kann insbesondere mittels einer Schweissverbindung, einer Klebeverbindung oder einer Pressverbindung derart auf dem zweiten Hülsenteilelement angebracht werden, dass eine Relativbewegung zwischen dem zweiten Hülsenteilelement und dem Halteelement ausgeschlossen ist. Das zweite Hülsenteilelement und das Halteelement gehen insbesondere eine starre Verbindung miteinander ein. Gemäss eines Ausführungsbeispiels können das Halteelement und das zweite Hülsenteilelement aus einem einzigen Stück bestehen.

Gemäss eines Ausführungsbeispiels enthält das zweite Hülsenteilelement ein Halteelement. Insbesondere ist das Halteelement an einem ersten Ende des zweiten Hülsenteilelements befestigt. Das Halteelement kann zur Aufnahme einer Haltevorrichtung ausgebildet sein. Die Haltevorrichtung kann mit dem Halteelement fest verbunden sein oder vom Halteelement abnehmbar sein. Die Haltevorrichtung kann beispielsweise zur Aufnahme eines Trackers ausgebildet sein. Der Tracker kann entweder starr mit der Befestigungsvorrichtung verbunden sein oder so konzipiert sein, dass er von der Haltevorrichtung gelöst und wieder angebracht werden kann, ohne dass sich die räumliche Position in Bezug auf die Haltevorrichtung ändert. Die Befestigungsvorrichtung ist so ausgebildet, dass sie eine stabile, ortsfeste Fixierung bietet, wenn der Tracker von der Haltevorrichtung abgenommen oder wieder an der Haltevorrichtung angebracht wird.

Die Erfindung kann in Kombination mit einem Navigationssystem verwendet werden, bei dem ein Tracker starr am Knochen befestigt werden muss. Das Navigationssystem kann insbesondere als ein chirurgisches Navigationssystem ausgebildet sein. Ein System enthaltend die Befestigungsvorrichtung und ein chirurgisches Navigationssystem kann vorzugsweise für Eingriffe am Kopf verwendet werden, z. B. für die Platzierung von Shunts, kraniale/Neuro-Navigation, Biopsien, HNO-Navigation oder endoskopische Navigation.

Die Erfindung bezieht sich auch auf eine Befestigungsvorrichtung für eine Therapievorrichtung, beispielsweise ein chirurgisches Hilfsmittel, beispielsweise ein Navigationshilfsmittel, insbesondere für einen Tracker. Die Befestigungsvorrichtung ist insbesondere dafür ausgelegt, starr in einer Öffnung im Knochen befestigt zu werden, wobei die Öffnung insbesondere ein vorgebohrtes Loch umfassen kann. Die Befestigungsvorrichtung ermöglicht insbesondere eine starre Befestigung einer Haltevorrichtung, beispielsweise für einen Tracker an einem Knochen für die chirurgische Navigation.

Ein Vorteil der erfindungsgemässen Befestigungsvorrichtung ist, dass kein zusätzliches Fixierungselement, wie beispielsweise eine Klammer oder eine Schraube, erforderlich ist. Die Befestigungsvorrichtung kann direkt an der Stelle des Eingriffs angebracht werden, was einen einfacheren und weniger invasiven Eingriff ermöglicht. Die Befestigungsvorrichtung kann derart ausgebildet sein, dass der Zugang zu Strukturen unterhalb der Öffnung möglich ist. Die Öffnung dient somit nicht nur der Aufnahme der Befestigungsvorrichtung, sondern auch zur Befestigung der Therapievorrichtung, insbesondere des chirurgischen Hilfsmittels. Die Befestigungsvorrichtung enthält gemäss des vorliegenden Ausführungsbeispiels auch eine Zugangsöffnung zu innerhalb des Körperteils liegenden Gewebe für eine therapeutische Behandlung. Insbesondere ist es vorteilhaft, wenn das Gewebe unterhalb der Öffnung nach dem Einbau der Befestigungsvorrichtung weiterhin für einen späteren chirurgischen Eingriff zugänglich ist. Ein derartiger chirurgischer Eingriff kann die Positionierung eines Mandrins, das Legen eines Katheters, eines Kabels oder einer Sonde durch die Öffnung in der Befestigungsvorrichtung umfassen, wenn diese in der Öffnung eingesetzt ist. Die für einen derartigen chirurgischen Eingriff erforderlichen Therapievorrichtungen haben in der Regel einen Durchmesser von weniger als 3 mm. Insbesondere kann der Innendurchmesser des zweiten Hülsenelements kann mindestens 3 mm betragen. Die in die Öffnung eingesetzte Befestigungsvorrichtung kann gemäss dieses Ausführungsbeispiels einer Therapievorrichtung den Zugang zu dem gewünschten Gewebe oder dem gewünschten anatomischen Bereich ermöglichen.

Die Befestigungsvorrichtung weist insbesondere ein Hülsenelement auf, welches vorteilhafterweise entsprechend dünnwandig sowie so kurz wie möglich ausgebildet ist, um in einem dünnen Knochen befestigt werden zu können und um Platz für eine Therapievorrichtung zu lassen, die durch die in die Öffnung eingesetzte Befestigungsvorrichtung hindurch geführt werden kann. Unter einem dünnen Knochen wird ein Knochen mit geringer Wandstärke verstanden, beispielsweise ein Schädelknochen.

Ein weiteres vorteilhaftes Merkmal der Befestigungsvorrichtung ist die Möglichkeit, dass die Befestigungsvorrichtung auch nachträglich angebracht oder entfernt werden kann, wenn sich eine Therapievorrichtung, wie beispielsweise ein Mandrin, ein Katheder, ein Kabel oder eine Sonde bereits in einer vorhanden Öffnung befindet oder nach einem Eingriff an Ort und Stelle verbleiben muss.

Gemäss eines Ausführungsbeispiels weist das erste Hülsenteilelement einen Längsschlitz auf. Der Längsschlitz erstreckt sich gemäss dieses Ausführungsbeispiels insbesondere von einem ersten Ende des ersten Hülsenteilelements zu einem zweiten Ende des ersten Hülsenteilelements. Insbesondere kann der Längsschlitz eine Schlitzbreite aufweisen, die grösser ist als der Durchmesser der Therapievorrichtung.

Gemäss eines Ausführungsbeispiels weist das zweite Hülsenteilelement einen Längsschlitz auf. Der Längsschlitz erstreckt sich gemäss dieses Ausführungsbeispiels insbesondere von einem ersten Ende des zweiten Hülsenteilelements zu einem zweiten Ende des zweiten Hülsenteilelements.

Insbesondere kann der Längsschlitz des ersten Hülsenteilelements und der Längsschlitz des zweiten Hülsenteilelements grösser sein oder vergrössert werden, sodass die Schlitzbreite der Längsschlitze grösser ist als der Durchmesser der Therapievorrichtung. Die Befestigungsvorrichtung kann gegebenenfalls in der Öffnung angebracht werden oder aus der Öffnung entfernt werden, ohne dass die Therapievorrichtung entfernt werden müsste. Der Längsschlitz des ersten Hülsenteilelements und der Längsschlitz des zweiten Hülsenteilelements sind hierzu übereinander liegend angeordnet. Das Hülsenelement, insbesondere in der Ausbildungsform als erstes Hülsenteilelement und als zweites Hülsenteilelement erstreckt sich somit nur über einen Teil des Umfangs der Öffnung. Mit anderen Worten nimmt das Hülsenelement somit nicht den vollen Umfang der Öffnung ein.

Die Befestigungsvorrichtung kann bei vielen chirurgischen Eingriffen verwendet werden, z. B., in der Neurochirurgie, bei der Platzierung von Shunts, in der HNO und in der Zahnchirurgie, insbesondere wenn die Führung einer Therapievorrichtung relativ zu einer Öffnung im Knochen gewünscht ist. Der chirurgische Eingriff kann entweder durch die Öffnung selbst oder auch in der Nähe der Öffnung in beliebiger Richtung erfolgen.

Ein an der Befestigungsvorrichtung angebrachtes Halteelement kann beispielsweise einen oder mehrere Teile eines Navigationssystems aufnehmen. Das Navigationssystem kann zumindest einen Tracker umfassen. Der Tracker kann beispielsweise als aktive oder passiver optischer Marker ausgebildet sein, der in Kombination mit einer Einzelkamera oder einem oder Stereokamerasystem verwendet werden kann. Ein Marker kann auch als Sensor für die elektromagnetische Navigation ausgebildet sein. Bei dem Tracker oder den Trackern kann es sich auch um integrierte oder inside-out Tracker handeln, oder Tracker, die Schattenbildtechnologie oder markerlose Navigationstechnologien nutzen.

Mittels eines Trackingsystems kann anhand der Position des am Knochen befestigten Trackers relativ zur Therapievorrichtung, die ebenfalls mit einem Tracker ausgestattet ist, die relative Position der Therapievorrichtung zur Anatomie bestimmt werden. Das Trackingsystem ist ein Bestandteil eines Navigationssystems, welches neben dem Trackingsystem eine Rechnereinheit für die Berechnung der räumlichen Beziehung des Trackers zu seinem Gegenstück erforderlich ist. Zum Beispiel kann ein an der Befestigungsvorrichtung angebrachter Tracker von einer Kamera verfolgt werden. Ein derartiger Tracker wird auch als Marker bezeichnet und kann passiv oder aktiv sein, wobei ein aktiver Tracker batteriebetrieben oder mit Kabeln verbunden sein kann.

Die Befestigungsvorrichtung kann für die Montage eines Trackers verwendet werden, der eine Kamera enthält oder als eine Kamera ausgebildet ist. Die Kamera ist Bestandteil eines Navigationssystems, wobei mittels des Navigationssystems eine Berechnung der räumlichen Position von Tracker und Kamera erfolgen kann. Insbesondere können miniaturisierte und entsprechend leichte Kameras eingesetzt werden, wenn ein auf Schattenbildtechnik basierendes Trackingsystem zum Einsatz kommt. Gemäss eines Ausführungsbeispiels enthält ein in einem auf Schattenbildtechnologie basierenden Trackingsystem eingesetzte Kamera einen optischen Sensor, der mit einem optischen Gitter abgedeckt ist. Das Navigationssystem kann die Position eines aktiven optischen Markers, der beispielsweise mit mehreren LEDs ausgestattet ist, auf der Grundlage des Schattens berechnen, der durch das optische Gitter auf den Bildsensor geworfen wird.

Mittels der Befestigungsvorrichtung kann ein Fiducial-Marker in Kombination mit dem Tracker verwendet werden oder dessen Befestigung ermöglicht werden. Dies ermöglicht bildbasierte chirurgische Eingriffe, bei denen der montierte oder integrierte Fiducial-Marker verwendet wird, um ein Bild wie einen C-Bogen, ISO-3D C-Bogen oder CT auf den Navigationskoordinatenrahmen zu registrieren.

Die beschriebene Befestigungsvorrichtung kann vorteilhafterweise in Kombination mit einem Navigationssystem verwendet werden. Das Navigationssystem umfasst das Trackingsystem wobei mittels des Navigationssystems auch die Position mindestens einer Therapievorrichtung mit einem Tracker in Bezug auf die Befestigungsvorrichtung messbar ist, einer Rechnereinheit zur Berechnung der Position der Therapievorrichtung auf der Grundlage der gemessenen Position des Trackers und einer Anzeigeeinheit insbesondere zur Anzeige von Navigationsinformation für den Chirurgen. Für die Anzeige der Navigationsinformation kann anstelle eines Monitors ein Augmented-Reality-Headset oder eine Anzeigeeinheit verwendet werden, welche eine Überlagerung der Navigationsinformation mit dem Operationsfeld ermöglicht.

Das Navigationssystem implementiert einen Arbeitsablauf für die chirurgische Navigation, der die Schritte der präoperativen Bildgebung und Planung, des Imports und der intraoperativen Registrierung des Plans und dann die Durchführung des Eingriffs unter Anleitung des Chirurgen umfassen kann.

Die Befestigungsvorrichtung gemäss der vorhergehenden Ausführungsbeispiele kann im sterilen Bereich eingesetzt werden. Dementsprechend kann die Befestigungsvorrichtung als Einweg- oder wiederverwendbare Befestigungsvorrichtung ausgebildet sein.

### Kurzbeschreibung der Zeichnungen

Nachfolgend werden die erfindungsgemässe Befestigungsvorrichtung anhand eines Ausführungsbeispiels dargestellt. Es zeigen
Fig. 1 eine Befestigungsvorrichtung gemäss eines exemplarischen Ausführungsbeispiels der Erfindung im Einbauzustand,
Fig. 2a eine Befestigungsvorrichtung gemäss eines ersten Ausführungsbeispiels in einer explosionsartigen Darstellung in einer ersten Stellung,
Fig. 2b die Befestigungsvorrichtung gemäss Fig. 2a im zusammengebauten Zustand in der ersten Stellung,
Fig. 3a eine Explosionsdarstellung der Befestigungsvorrichtung ohne Haltevorrichtung,
Fig. 3b eine Ansicht der Befestigungsvorrichtung gemäss Fig. 2b in einer zweiten Stellung,
Fig. 4a eine perspektivische Ansicht der Befestigungsvorrichtung mit dem Halteelement in einer ersten Stellung,
Fig. 4b eine weitere perspektivische Ansicht der Befestigungsvorrichtung mit dem Halteelement in der ersten Stellung,
Fig. 5a eine perspektivische Ansicht der Befestigungsvorrichtung mit dem Halteelement in einer zweiten Stellung,
Fig. 5b eine weitere perspektivische Ansicht der Befestigungsvorrichtung mit dem Halteelement in der zweiten Stellung,
Fig. 6a einen Radialschnitt durch die Befestigungsvorrichtung gemäss Fig. 2a in der ersten Stellung,
Fig. 6b einen Radialschnitt durch die Befestigungsvorrichtung gemäss Fig. 3b in der zweiten Stellung,
Fig. 7a eine perspektivische Ansicht einer Befestigungsvorrichtung gemäss eines zweiten Ausführungsbeispiels mit dem Halteelement in einer ersten Stellung,
Fig. 7b eine weitere perspektivische Ansicht der Befestigungsvorrichtung gemäss Fig. 7a mit dem Halteelement in der ersten Stellung,
Fig. 8a eine perspektivische Ansicht der Befestigungsvorrichtung gemäss des zweiten Ausführungsbeispiels mit dem Halteelement in der zweiten Stellung,
Fig. 8b eine weitere perspektivische Ansicht der Befestigungsvorrichtung mit dem Halteelement in der zweiten Stellung,
Fig. 9 ein Anwendungsbeispiel der erfindungsgemässen Befestigungsvorrichtung nach einem der Ausführungsbeispiele.

### Detaillierte Beschreibung der Zeichnungen

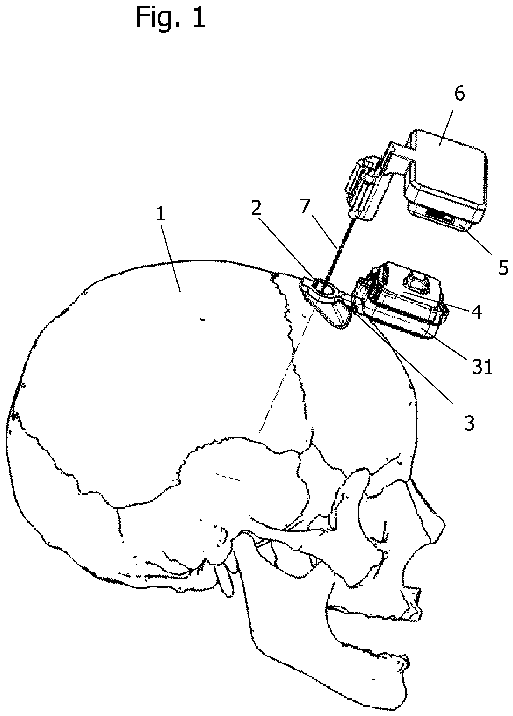

In Fig. 1 ist ein Ausführungsbeispiel einer Befestigungsvorrichtung 2 für eine Trackeranordnung sowie zur Führung einer Therapievorrichtung 6, insbesondere eines chirurgischen Instruments, beispielsweise eines Mandrins 7 zur Shuntplatzierung, durch eine in Fig. 1 nicht sichtbare Öffnung 9 in einem Schädelknochen 1 dargestellt. Diese Öffnung 9 kann insbesondere als Bohrloch ausgebildet sein. Die Befestigungsvorrichtung 2 ist derart in der Öffnung aufgenommen, dass sie die Öffnung 9 im eingebauten Zustand allenfalls unwesentlich überragt.

Die Trackeranordnung umfasst einen ersten Tracker 4 und einen zweiten Tracker 5. Der erste Tracker 4 kann insbesondere einen optischen Marker enthalten oder als optischer Marker ausgebildet sein. Der zweite Tracker 5 kann eine Kamera enthalten. Der erste Tracker 4 ist mittels der Befestigungsvorrichtung 2 starr am Schädelknochen 1 befestigt, sodass keine zusätzlichen Schnitte oder Fixierungen am Schädelknochen 1 vorgesehen werden müssen.

Der erste Tracker 4 ist einem Halteelement 3 aufgenommen, welches mit der Befestigungsvorrichtung 2 verbindbar ist. Das Halteelement 3 kann somit von der Befestigungsvorrichtung 2 abgenommen werden. Gemäss des vorliegenden Ausführungsbeispiels ist der zweite Tracker 5 mit der Therapievorrichtung 6 verbunden. Die Therapievorrichtung 6 ist somit gemäss des vorliegenden Ausführungsbeispiels mit dem zweiten Tracker 5 verbunden und kann in Bezug auf die den im Halteelement 3 aufgenommenen ersten Tracker 4 navigiert werden. Da das Halteelement 3 mit der Befestigungsvorrichtung 2 in einer definierten Position relativ zu der Öffnung anbringbar ist, kann somit über die hiermit definierte Position des ersten Trackers 4 auch dessen Position auf dem Schädelknochen 1 und das Weichteilgewebe unterhalb des Schädelknochens 1 bestimmt werden.

Gemäss des vorliegenden Ausführungsbeispiels wird die Position des ersten Trackers 4 relativ zum zweiten Tracker 5 ermittelt. Wenn der erste Tracker 4 einen optischen Marker enthält, kann dessen 6DOF-Position relativ zu dem zweiten Tracker 5 bzw. dessen Kamera gemessen werden. Alternativ kann der zweite Tracker 5 ebenfalls einen optischen Marker enthalten, wobei eine nicht zur Trackeranordnung gehörige externe Kamera verwendet werden kann, um die 6DOF Position des ersten Trackers 4 relativ zum zweiten Tracker 5 zu ermitteln. Gemäss eines weiteren Ausführungsbeispiels können die Therapievorrichtung 6 und die Befestigungsvorrichtung 2 optische Marker enthalten, sodass eine Ermittlung der 6DOF-Position der Befestigungsvorrichtung 2 und der Therapievorrichtung 6 ermöglicht ist.

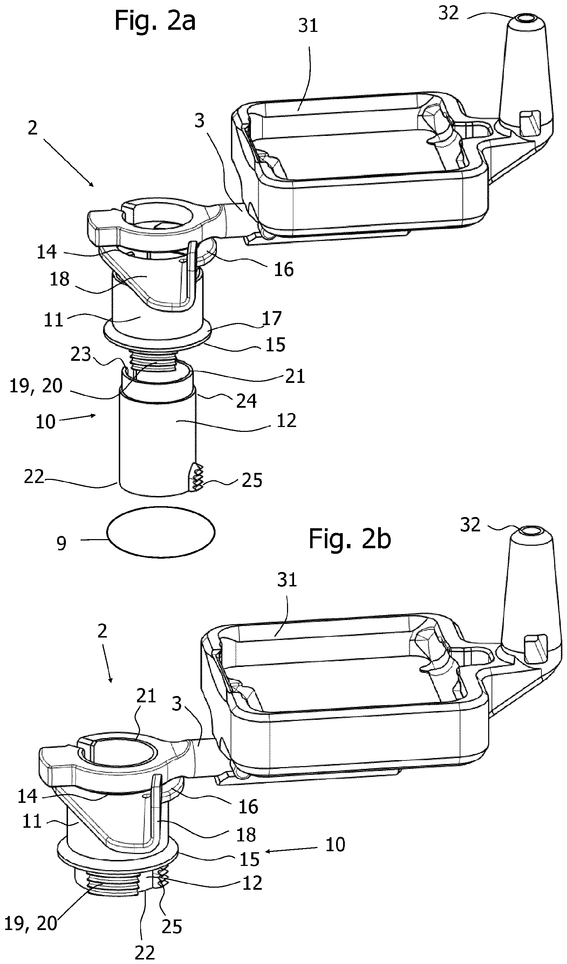

In Fig. 2a ist die Befestigungsvorrichtung 2, die in der Öffnung 9 aufgenommen werden soll, gezeigt. Die Befestigungsvorrichtung 2 enthält ein Hülsenelement 10, welches zur Aufnahme in der Öffnung 9 ausgebildet ist. Das Hülsenelement 10 enthält ein erstes Hülsenteilelement 11 und ein zweites Hülsenteilelement 12. Fig. 2a zeigt eine explosionsartige Darstellung der Befestigungsvorrichtung 2, in Fig. 2b ist die Befestigungsvorrichtung 2 im zusammengebauten Zustand dargestellt. Im zusammengebauten Zustand ist das erste Hülsenteilelement 11 über das zweite Hülsenteilelement 12 gestülpt. Das erste Hülsenteilelement 11 ist relativ zum zweiten Hülsenteilelement 12 drehbar. Insbesondere ist das erste Hülsenteilelement 11 um das zweite Hülsenteilelement 12 drehbar.

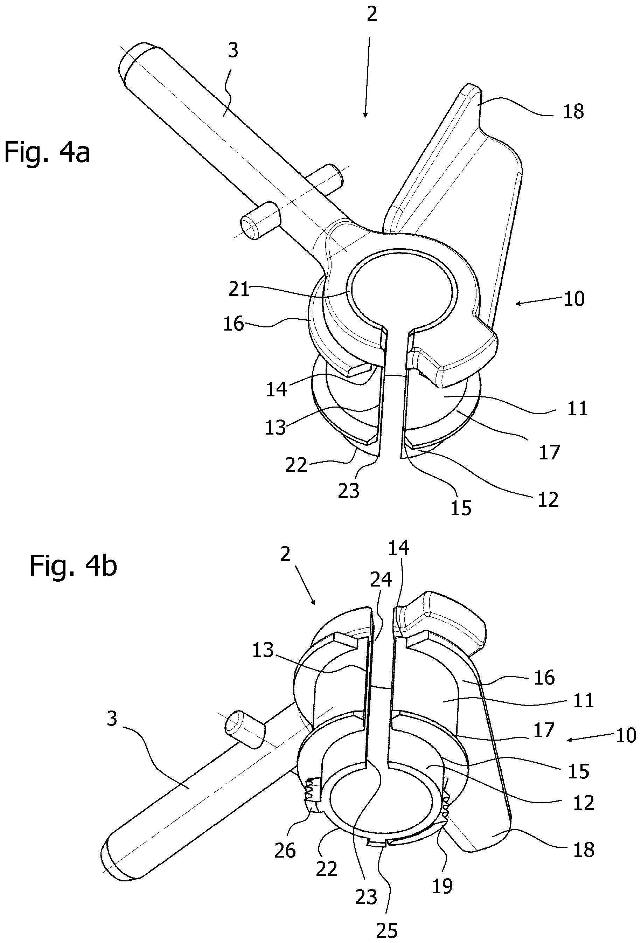

Das erste Hülsenteilelement 11 ist gemäss des vorliegenden Ausführungsbeispiels als ein zylinderförmiger Hohlkörper ausgebildet, welcher einen Längsschlitz 13 aufweist, was in Fig. 4a oder Fig. 4b gezeigt ist. Der Längsschlitz 13 erstreckt sich von einem ersten Ende 14 des ersten Hülsenteilelements 11 zu einem zweiten Ende 15 des ersten Hülsenteilelements 11. Das erste Ende 14 des ersten Hülsenteilelements 11 umfasst einen ersten Flansch 16 sowie ein Griffelement 18. Das zweite Ende 15 umfasst einen zweiten Flansch 17 und einen den zweiten Flansch 17 in Richtung der Längsachse des zylinderförmigen Hohlkörpers überragenden Fortsatz 19. Der Fortsatz 19 erstreckt sich über einen Teil des Umfangs des ersten Hülsenteilelements 11. Gemäss des vorliegenden Ausführungsbeispiels wird durch den Fortsatz 19 ein schalenförmiger Vorsprung ausgebildet. Der Fortsatz 19 weist insbesondere einen Krümmungsradius auf, der dem Innendurchmesser des zylinderförmigen Hohlkörpers entspricht. Der Fortsatz 19 weist gemäss des vorliegenden Ausführungsbeispiels eine Segmentlänge auf, die im Bereich von 10% bis einschliesslich 40% des Umfangs des zylinderförmigen Hohlkörpers liegt. Der Fortsatz 19 weist eine axiale Länge auf, die gemäss des vorliegenden Ausführungsbeispiels im Bereich von 5% bis einschliesslich 50% des zwischen dem ersten Flansch 16 und dem zweiten Flansch 17 in Richtung der Längsachse gemessenen Abstandes liegt.

Der Fortsatz 19 weist gemäss des vorliegenden Ausführungsbeispiels eine Profilierung 20 auf der Aussenseite auf. Die Profilierung kann mindestens ein Element aus der Gruppe bestehend aus Rippen, Rillen, Zähnen, Spikes, Spitzen, Schneidelementen oder Gewinden umfassen. Die Profilierung kann insbesondere Rippen oder Rillen umfassen. Die Rippen oder Rillen erstrecken sich gemäss des vorliegenden Ausführungsbeispiels in im Wesentlichen radialer Richtung. Mittels der Profilierung wird eine raue Oberfläche erzeugt, welche einen verbesserten Halt in der Öffnung 9 bietet. Mittels der Profilierung wird die Reibung zwischen der Innenwand der Öffnung 9 und dem ersten Hülsenteilelement 11 erhöht, sodass das erste Hülsenteilelement 11 sich im Einbauzustand in der Öffnung 9 verkeilen kann. Alternativ kann die Profilierung derart ausgebildet sein, dass sie in die Innenwand der Öffnung eingreifen kann.

Das zweite Hülsenteilelement 12 ist gemäss des vorliegenden Ausführungsbeispiels als ein zylinderförmiger Hohlkörper ausgebildet, welcher einen Längsschlitz 23 aufweist, was in Fig. 4a oder Fig. 4b gezeigt ist. Der Längsschlitz 23 erstreckt sich von einem ersten Ende 21 des zweiten Hülsenteilelements 12 zu einem zweiten Ende 22 des Hülsenteilelements 12. Gemäss des vorliegenden Ausführungsbeispiels weist das zweite Hülsenteilelement 12 eine Innenseite mit glatter Oberfläche auf. Insbesondere enthält die Innenseite des zweiten Hülsenteilelements 12 keine Unebenheiten, Vorsprünge oder Querschnittsänderungen. Durch den von der Innenseite des zweiten Hülsenteilelements 12 ausgebildeten Innenraum kann eine Therapievorrichtung hindurchgeführt werden, wie beispielsweise ein chirurgisches Instrument, ein Messgerät, eine Kanüle, ein Schlauch. Gemäss des vorliegenden Ausführungsbeispiels weist das zweite Hülsenteilelement 12 eine Aussenseite auf, die zumindest einen Absatz 24. Gemäss des vorliegenden Ausführungsbeispiel enthält das zweite Hülsenteilelement 12 eine Vorsprunganordnung, umfassend zumindest einen Vorsprung 25, 26. Der Absatz 24 ist näher zum ersten Ende 21 gelegen als zum zweiten Ende 22. Der Absatz 24 dient der Auflage des Halteelements 3, welches im eingebauten Zustand, wie in Fig. 2b, Fig. 4a oder Fig. 4b gezeigt, auf dem Absatz 24 aufliegt. Das Halteelement 3 ist mit dem zweiten Hülsenteilelement 12 fest verbunden, beispielsweise verklebt, verschweisst oder verschraubt. Alternativ können das Halteelement 3 und das zweite Hülsenteilelement 12 aus einem einzigen Bauteil ausgebildet sein.

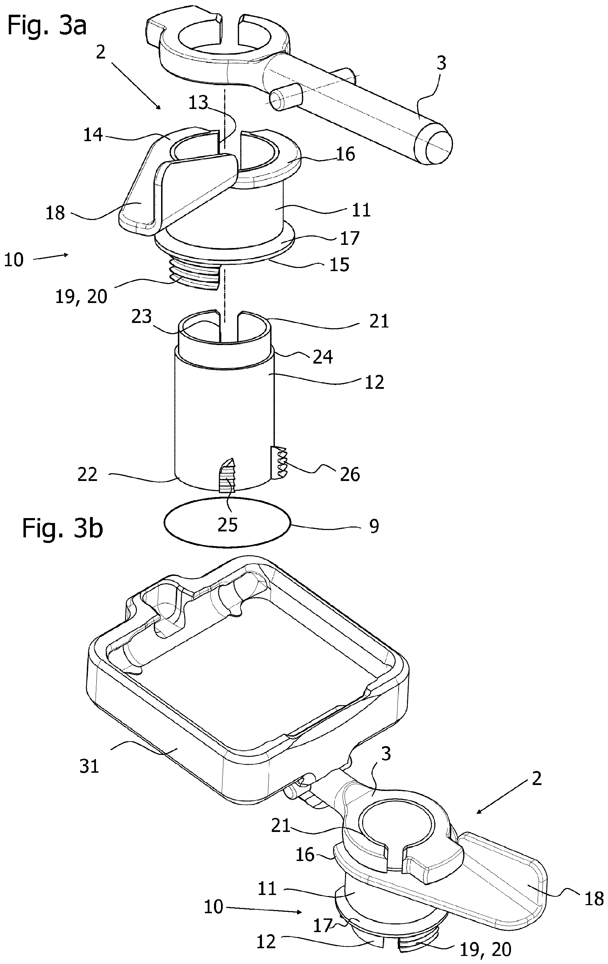

Gemäss des vorliegenden Ausführungsbeispiels enthält das zweite Hülsenteilelement 12 einen ersten Vorsprung 25 und einen zweiten Vorsprung 26. In Fig. 2a und Fig. 2b ist nur der erste Vorsprung 25 sichtbar, in Fig. 3a sind der erste Vorsprung 25 und der zweite Vorsprung 26 gezeigt. Zumindest einer der ersten Vorsprünge 25 und der zweiten Vorsprünge 26 kann eine Profilierung beispielsweise in Form von Rippen, Rillen, Zähnen, Spikes oder Gewinden aufweisen. Der erste und der zweite Vorsprung 25, 26 sind im eingebauten Zustand in Kontakt mit der Innenwand der Öffnung 9 und können eine Kontaktfläche mit der Innenwand der Öffnung 9 ausbilden. Mittels einer Profilierung kann an den Kontaktflächen eine stabilere Fixierung der Befestigungsvorrichtung 2 in der Öffnung 9, beispielsweise in dem Bohrloch im Körperteil, erzielt werden.

Insbesondere kann die Profilierung als eine Mehrzahl von Rillen oder Rippen ausgebildet sein, die in radialer Richtung verlaufen. Die Segmentlänge des entsprechenden ersten oder zweiten Vorsprungs 25, 26 kann insbesondere maximal 10% des Umfangs des zweiten Hülsenteilelements 12 an dessen zweiten Ende 22 betragen. Der entsprechende erste oder zweite Vorsprung 25, 26 kann in axialer Richtung maximal 30% der Gesamtlänge des zweiten Hülsenteilelements 12 betragen. Mit Gesamtlänge ist der Abstand in axialer Richtung vom ersten Ende 21 bis zum zweiten Ende 22 gemeint.

Da gemäss des vorliegenden Ausführungsbeispiels sowohl das erste Hülsenteilelement 11 als auch das zweite Hülsenteilelement 12 je einen Längsschlitz 13, 23 aufweisen, ist es einfach möglich, die ersten und zweiten Hülsenteilelemente 11, 12 mittels eines Werkzeugs zusammenzudrücken, um das Einführen der Befestigungsvorrichtung 2 in die Öffnung 9 zu erleichtern. Ein solcher Längsschlitz 13, 23 ist auch vorteilhaft, um die Befestigungsvorrichtung 2 später wieder aus der Öffnung 9 zu entfernen, selbst wenn eine durch die Öffnung 9 und durch die Befestigungsvorrichtung 2 eingeführte Therapievorrichtung 6 noch an Ort und Stelle verbleiben soll. Zum Zusammendrücken der Befestigungsvorrichtung 2 kann ein Einführungsinstrument oder eine Zange verwendet werden.

Das Halteelement 3 kann zur Aufnahme einer Haltevorrichtung 31 ausgebildet sein. Die Haltevorrichtung 31 kann beispielsweise zur Aufnahme eines Trackers ausgebildet sein, insbesondere des in Fig. 1 dargestellten ersten Trackers 4. Die Haltevorrichtung 31 kann vom Halteelement 3 abnehmbar sein und ist derart ausgebildet, dass sie an einer vorbestimmten räumlichen Position abgenommen werden kann und auch in dieser vorbestimmten räumlichen Position wieder angebracht werden kann. Gemäss des vorliegenden Ausführungsbeispiels enthält die Haltevorrichtung 31 ein Kalibrierungselement 32. Das Kalibrierungselement 32 ist ausgebildet, um eine Kalibrierung oder Registrierung einer Therapievorrichtung zu ermöglichen, beispielsweise der in Fig. 1 gezeigten Therapievorrichtung 6. Beispielsweise kann das Kalibrierungselement 32 eine Kalibrierungsöffnung oder einen Kalibrierungskanal umfassen, in welchen ein als Spitzenelement ausgebildetes Gegenstück der Therapievorrichtung einführbar ist.

Fig. 2b zeigt die Befestigungsvorrichtung 2 im zusammengebauten Zustand. Das erste Hülsenteilelement 11 enthält ein Griffelement 18, welches mit dem ersten Hülsenteilelement 11 fest verbunden ist. Mit anderen Worten wird durch Betätigung des Griffelements 18 eine Drehung des ersten Hülsenteilelements 11 relativ zum zweiten Hülsenteilelement 12 ausgelöst. Das Griffelement 18 ist insbesondere zur manuellen Betätigung ausgebildet. Das Griffelement 18 kann gemäss des vorliegenden Ausführungsbeispiels zwischen der in Fig. 2a, Fig. 2b, Fig. 3a, Fig. 4a, Fig. 4b, Fig. 6a dargestellten ersten Stellung und einer in Fig. 3b, Fig. 5a, Fig. 5b, Fig. 6b gezeigten zweiten Stellung bewegt werden. In der in Fig. 2b dargestellten Darstellung ist die Befestigungsvorrichtung 2 im Einbauzustand gezeigt. Im Einbauzustand ist die Befestigungsvorrichtung 2 in der Öffnung 9 positioniert. Die Öffnung 9 ist in der vorliegenden Darstellung weggelassen, um den Blick auf die im Inneren der Öffnung 9 befindlichen Bauteile freizugeben.

Die in Fig. 2b dargestellte Befestigungsvorrichtung 2 ist in ihrer ersten Stellung gezeigt, in welcher der Einbau der Befestigungsvorrichtung 2 in die Öffnung 9 oder der Ausbau der Befestigungsvorrichtung 2 aus der Öffnung 9 ermöglicht ist. Beim Einbau wird das Hülsenelement 10 derart in der Öffnung 9 positioniert, dass der Flansch 16 bzw. das Griffelement 18 auf der äusseren Oberfläche des die Öffnung 9 enthaltenden Körperteils, insbesondere einer Gewebeschicht, welche einen Schädelknochens 1 bedeckt, aufliegt. Um das Einführen des Hülsenelements 10 in die Öffnung 9 zu erleichtern, kann eine Klemmkraft auf die beidseitig der Längsschlitze 13, 23 liegenden Mantelteile des ersten und zweiten Hülsenteilelements 11, 12 ausgeübt werden, die bewirkt, dass sich die Schlitzbreite der Längsschlitze 13, 23 verringert. Die Längsschlitze 13, 23 fluchten in der in Fig. 2b gezeigten ersten Stellung, sodass eine besonders kraftsparende Verringerung der Querschnittsfläche des Hülsenelements 10 erzielbar ist.

In der dargestellten ersten Stellung liegen die ersten und zweiten Vorsprünge 25, 26 des zweiten Hülsenteilelements 12 angrenzend an oder in der Nähe des Fortsatzes 19 des ersten Hülsenteilelements 11, was in Fig. 4b besonders gut sichtbar ist. Die Vorsprünge 25, 26 und der Fortsatz 19 schliessen zueinander einen Winkel in einer rechtwinklig zur Längsachse angeordneten Ebene ein, der nicht grösser als 200 Grad ist, insbesondere nicht grösser als 180 Grad ist. In der ersten Stellung ist somit die Querschnittsfläche des zweiten Hülsenteilelement 12 mit den ersten und zweiten Vorsprüngen 25, 26 sowie des Fortsatzes 19 des ersten Hülsenteilelements 11 maximal gleich gross wie die Querschnittsfläche des Flansches 17 des ersten Hülsenteilelements 11. In der ersten Stellung ist somit das Befestigungselement 2 in die Öffnung 9 einführbar, da die Öffnung 9 eine Querschnittsfläche aufweist, die etwas grösser als die Querschnittsfläche des Flansches 17 des ersten Hülsenteilelements 11 ist.

Fig. 3a zeigt eine Explosionsdarstellung der Befestigungsvorrichtung 2 ohne Tracker, wobei die Ansicht gemäss Fig. 3a um einen Winkel von ca. 20 bis 40 Grad versetzt in Bezug auf die Darstellung gemäss Fig. 2a ist. Auch in Fig. 3a ist die Befestigungsvorrichtung 2 in der ersten Stellung gezeigt. In Fig. 3a ist deutlicher sichtbar, dass der Längsschlitz 13 des ersten Hülsenteilelements 11 mit dem Längsschlitz 23 des zweiten Hülsenteilelements 12 fluchtet. Zudem sind der erste Vorsprung 25 und der zweite Vorsprung 26 des zweiten Hülsenteilelements 12 sichtbar.

Fig. 3b zeigt eine Ansicht der Befestigungsvorrichtung 2 mit einer Haltevorrichtung 31 für einen Tracker in der zweiten Stellung. In der zweiten Stellung ist die Befestigungsvorrichtung 2 in der Öffnung 9 aufgenommen und fixiert. Die Öffnung 9 ist in der vorliegenden Darstellung weggelassen, um die Position der Bauelemente besser zu verdeutlichen. Die zweite Stellung wird aus der in Fig. 2b gezeigten ersten Stellung erhalten, indem man die in der Öffnung 9 befindliche Befestigungsvorrichtung 2 gemäss Fig. 2b fixiert. Die Fixierung der Befestigungsvorrichtung 2 in der Öffnung erfolgt durch die Betätigung des Griffelements 18, welches gegenüber der in Fig. 2b gezeigten Position um maximal 90 Grad um die gemeinsame Längsachse des Hülsenelements 10 gedreht ist. Das Griffelement 18 ist fest mit dem ersten Hülsenteilelement 11 verbunden, sodass bei einer Drehbewegung des Griffelements 18 das erste Hülsenteilelement 11 relativ zum zweiten Hülsenteilelement 12 bewegbar ist.

In Fig. 3b ist der Längsschlitz 23 des zweiten Hülsenteilelements 12 noch sichtbar, der Längsschlitz 13 des ersten Hülsenteilelements 11 jedoch unsichtbar. Mit der Drehbewegung des ersten Hülsenteilelements 11 relativ zum zweiten Hülsenteilelement 12 verändert sich auch die Position des mit dem ersten Hülsenteilelements 11 verbundenen Fortsatzes 19. Der Fortsatz 19 befindet sich in der zweiten Stellung in einer in Bezug auf die ersten und zweiten Vorsprünge 25, 26 des zweiten Hülsenteilelements 12 gegenüberliegenden Position. In der zweiten Stellung sind sowohl die ersten und zweiten Vorsprünge 25, 26 als auch der Fortsatz 19 in Kontakt mit der Innenwand der Öffnung 9. In der zweiten Stellung ist die Befestigungsvorrichtung 2 in der Öffnung 9 derart befestigt, dass ein Verrutschen des Halteelements 3 und einer allfälligen, am Halteelement 3 befestigten Haltevorrichtung 31 ausgeschlossen ist. Insbesondere kann eine auf der äusseren Oberfläche des Fortsatzes 19 befindliche Profilierung 20 zu einer weiteren Erhöhung des Reibungswiderstandes dienen. Wenn die Profilierung 20 scharfkantige Elemente, beispielsweise Rippen, Rillen, Zähne, Spikes, Spitzen, Schneidelemente oder Gewinde enthält, kann sogar ein Eingriff der Profilierung 20 in die Innenwand der Öffnung 9 erfolgen, sodass ein fester Sitz der Befestigungsvorrichtung 2 in der Öffnung 9 gewährleistet werden kann. Mittels des Flansches 17 des ersten Hülsenteilelements 11 kann eine verbesserte Zentrierung und Auflage der Befestigungsvorrichtung 2 während der Montage derselben in der Öffnung 9 erfolgen.

Fig. 4a zeigt eine perspektivische Ansicht der Befestigungsvorrichtung 2 und des Halteelements 3 in der ersten Stellung. Das Halteelement 3 ist fest mit dem zweiten Hülsenteilelement 12 verbunden. Gemäss des vorliegenden Ausführungsbeispiels bildet das erste Ende 21 des zweiten Hülsenteilelements 12 eine gemeinsame Oberfläche mit dem Halteelement 3 aus, im Idealfall ist diese Verbindung spaltfrei ausgebildet, sodass eine einfache Reinigung der Befestigungsvorrichtung 2 ermöglicht ist. Selbstverständlich können das zweite Hülsenteilelement 12 und das Halteelement 3 auch als ein einziges Bauteil ausgebildet sein. Das erste Hülsenteilelement 11 kann über das zweite Hülsenteilelement 12 gestülpt werden, bis es an der Unterseite des Halteelements 3 anliegt. Gemäss des vorliegenden Ausführungsbeispiels weist der Flansch 16, der am ersten Ende 14 des ersten Hülsenteilelements 11 einen grösseren Aussendurchmesser auf als das Halteelement 3, sodass der Flansch 16 das Halteelement 3 mehrheitlich überragt. Das Halteelement 3 ist als ein stangenförmiger Körper mit einer ringförmigen Erweiterung ausgebildet, welche zur Aufnahme auf dem zweiten Halteelement 12 ausgebildet ist. Die ringförmige Erweiterung enthält eine Verdickung, die gemäss des vorliegenden Ausführungsbeispiels gegenüberliegend zum stangenförmigen Körper angeordnet ist. Diese Verdickung dient als Anschlag für das Griffelement 18.

Fig. 4b zeigt eine perspektivische Ansicht der Befestigungsvorrichtung 2 und des Halteelements 3 in der ersten Stellung, wobei in dieser Ansicht das zweite Ende 22 des zweiten Hülsenteilelements 12 sowie das zweite Ende 15 des ersten Hülsenteilelements 11 sichtbar sind. In Fig. 4b sind auch der Fortsatz 19 des ersten Hülsenteilelements 11 und der erste Vorsprung 25 und der zweite Vorsprung 26 des zweiten Hülsenteilelements 12 gezeigt. In Fig. 4b ist auch deutlich zu erkennen, dass in der ersten Stellung die ersten und zweiten Vorsprünge 25, 26 und der Fortsatz 19 ungefähr die Hälfte des Umfangs des zweiten Hülsenteilelements 12 einnehmen. In dieser ersten Stellung weist somit die Befestigungsvorrichtung 2 eine minimale Querschnittsfläche auf, was deren Einführung in die Öffnung 9 besonders einfach macht. Zudem ist es möglich, mittels eines Werkzeugs die Schlitzbreite des Längsschlitzes 13 des ersten Hülsenteilelements 11 und des Längsschlitzes 23 des zweiten Hülsenteilelements 12 zu verringern, sodass das Befestigungselement 2 einfacher in die Öffnung 9 eingeführt werden kann.

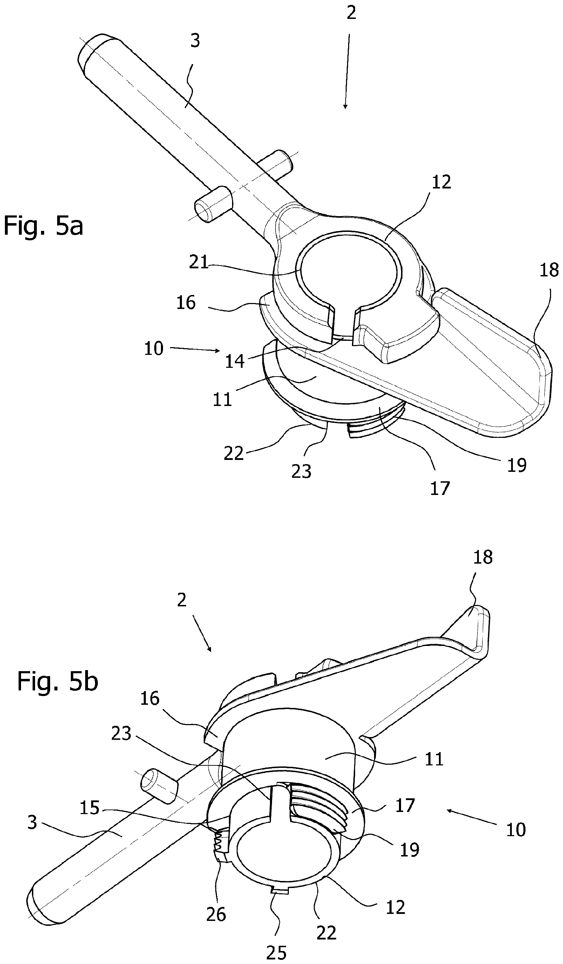

Fig. 5a zeigt eine perspektivische Ansicht der Befestigungsvorrichtung 2 und des Halteelements 3 in der zweiten Stellung, gemäss welcher das erste Hülsenteilelement 11 relativ zum zweiten Hülsenteilelement 12 um einen Winkel von ungefähr 90 Grad verdreht worden ist. Das Griffelement 18 erstreckt sich gegenüberliegend zum stangenförmigen Körper des Halteelements 3.

Fig. 5b zeigt eine perspektivische Ansicht der Befestigungsvorrichtung 2 und des Halteelements 3 in der zweiten Stellung, wobei in dieser Ansicht das zweite Ende 22 des zweiten Hülsenteilelements 12 sowie das zweite Ende 15 des ersten Hülsenteilelements 11 sichtbar sind. In Fig. 5b sind auch der Fortsatz 19 des ersten Hülsenteilelements 11 und der erste Vorsprung 25 und der zweite Vorsprung 26 des zweiten Hülsenteilelements 12 gezeigt. In Fig. 5b ist auch deutlich zu erkennen, dass in der zweiten Stellung die ersten und zweiten Vorsprünge 25, 26 und der Fortsatz 19 ungefähr gegenüberliegend zueinander angeordnet sind. Die Vorsprünge 25, 26 und der Fortsatz 19 sind derart angeordnet, dass sie sie eine Klemmwirkung entfalten, sodass die Befestigungsvorrichtung 2 in der Öffnung 9 fixiert ist. In dieser zweiten Stellung weist somit die Befestigungsvorrichtung 2 eine maximale Querschnittsfläche auf, was einen optimalen Halt in der Öffnung 9 gewährleistet. Zudem ist es möglich, dass der Fortsatz 19 eine Profilierung aufweist, die in die Innenwand der Öffnung 9 eingreift, sodass die Befestigungsvorrichtung in der zweiten Stellung arretiert ist. Auch die ersten und zweiten Vorsprünge können Profilierungen aufweisen, mittels welchen zumindest der Reibungswiderstand an der Innenwand der Öffnung erhöht werden kann. Insbesondere können die Profilierungen des ersten und zweiten Vorsprungs 25, 26 derart ausgebildet sein, dass sie in die Innenwand der Öffnung 9 eingreifen können. Die Position des ersten Hülsenteilelements 11 relativ zum zweiten Hülsenteilelement 12 in der zweiten Stellung ist abhängig vom Innendurchmesser der Öffnung 9. Die Öffnung 9 kann einen effektiven Durchmesser aufweisen, der vom Nenndurchmesser geringfügig abweicht. Wenn der Nenndurchmesser der Öffnung 9 beispielsweise im Bereich von 10 bis einschliesslich 15 mm liegt, kann der effektive Durchmesser eine Abweichung von bis zu 1 mm in Bezug auf den Nenndurchmesser aufweisen. Die Position des ersten Hülsenteilelements 11 in der zweiten Stellung ist einstellbar, sodass Abweichungen zwischen dem Nenndurchmesser und dem effektiven Durchmesser der Öffnung 9 durch die Befestigungsvorrichtung ausgeglichen werden können. Die Position des Fortsatzes 19 relativ zu den ersten und zweiten Vorsprüngen 25, 26 kann somit in der zweiten Stellung in Abhängigkeit vom effektiven Durchmesser der Öffnung 9 unterschiedlich eingestellt sein.

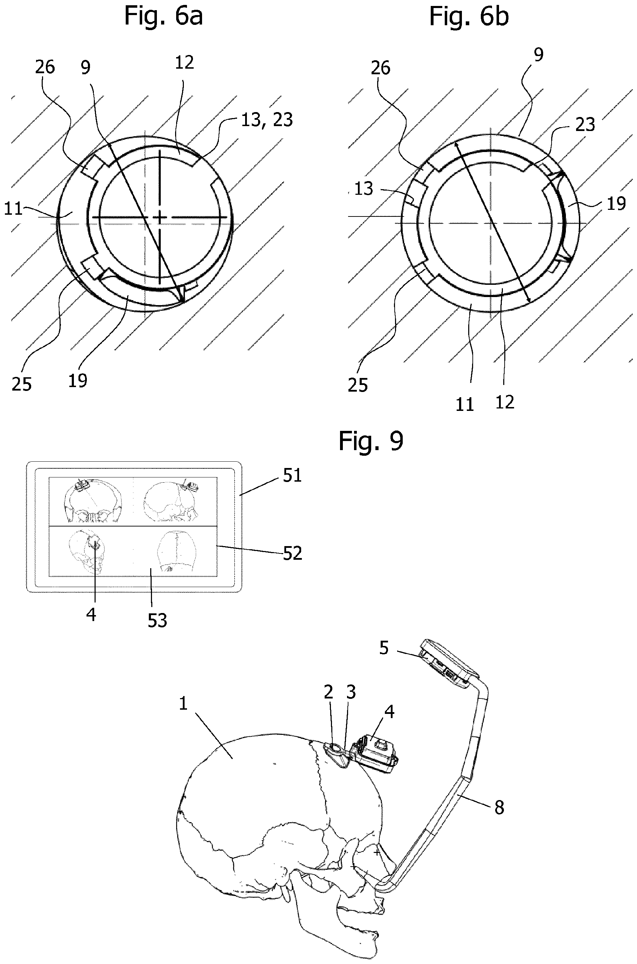

Fig. 6a zeigt einen Radialschnitt durch die Befestigungsvorrichtung 2 gemäss Fig. 2a in der ersten Stellung, welche in eine im Schnitt dargestellte Öffnung 9 eingeführt wird. Die Befestigungsvorrichtung 2 wird vorteilhafterweise nicht koaxial zur Mittenachse der Öffnung 9 eingeschoben, sondern mit einem Neigungswinkel, sodass der Fortsatz 19 des ersten Hülsenteilelements 11 sowie die Vorsprünge 25 und 26 des zweiten Hülsenteilelements beim Einführen der Befestigungsvorrichtung 2 in die Öffnung 9 nicht mit deren Innenwand in Kontakt kommen.

Fig. 6b zeigt einen Radialschnitt durch die Befestigungsvorrichtung 2 gemäss Fig. 3b in der zweiten Stellung, die sich in der Öffnung 9 befindet.

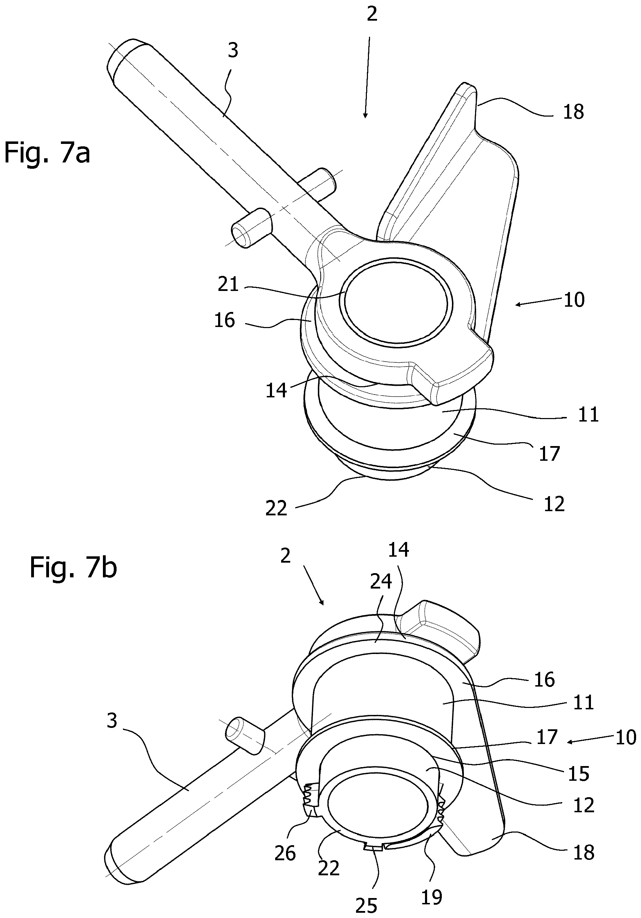

Fig. 7a zeigt eine perspektivische Ansicht einer Befestigungsvorrichtung 2 gemäss eines zweiten Ausführungsbeispiels mit dem Halteelement 3 in einer ersten Stellung. Für dieses Ausführungsbeispiel werden für gleiche oder gleichwirkende Bauteile dieselben Bezugszeichen wie für das erste Ausführungsbeispiel verwendet. Gemäss des zweiten Ausführungsbeispiels enthalten das erste Hülsenteilelement 11 und das zweite Hülsenteilelement 12 keinen Längsschlitz. Das Halteelement 3 ist fest mit dem zweiten Hülsenteilelement 12 verbunden. Gemäss des vorliegenden Ausführungsbeispiels bildet das erste Ende 21 des zweiten Hülsenteilelements 12 eine gemeinsame Oberfläche mit dem Halteelement 3 aus, im Idealfall ist diese Verbindung spaltfrei ausgebildet, sodass eine einfache Reinigung der Befestigungsvorrichtung 2 ermöglicht ist. Selbstverständlich können das zweite Hülsenteilelement 12 und das Halteelement 3 auch als ein einziges Bauteil ausgebildet sein. Das erste Hülsenteilelement 11 kann über das zweite Hülsenteilelement 12 gestülpt werden, bis es an der Unterseite des Halteelements 3 anliegt. Gemäss des vorliegenden Ausführungsbeispiels weist der Flansch 16, der am ersten Ende 14 des ersten Hülsenteilelements 11 einen grösseren Aussendurchmesser auf als das Halteelement 3, sodass der Flansch 16 das Halteelement 3 mehrheitlich überragt. Das Halteelement 3 ist als ein stangenförmiger Körper mit einer ringförmigen Erweiterung ausgebildet, welche zur Aufnahme auf dem zweiten Halteelement 12 ausgebildet ist. Die ringförmige Erweiterung enthält eine Verdickung, die gemäss des vorliegenden Ausführungsbeispiels gegenüberliegend zum stangenförmigen Körper angeordnet ist. Diese Verdickung dient als Anschlag für das Griffelement 18.

Fig. 7b zeigt eine weitere perspektivische Ansicht der Befestigungsvorrichtung gemäss Fig. 7a mit dem Halteelement 3 in der ersten Stellung, wobei in dieser Ansicht das zweite Ende 22 des zweiten Hülsenteilelements 12 sowie das zweite Ende 15 des ersten Hülsenteilelements 11 sichtbar sind. In Fig. 7b sind auch der Fortsatz 19 des ersten Hülsenteilelements 11 und der erste Vorsprung 25 und der zweite Vorsprung 26 des zweiten Hülsenteilelements 12 gezeigt. In Fig. 7b ist auch deutlich zu erkennen, dass in der ersten Stellung die ersten und zweiten Vorsprünge 25, 26 und der Fortsatz 19 ungefähr die Hälfte des Umfangs des zweiten Hülsenteilelements 12 einnehmen. In dieser ersten

Stellung weist somit die Befestigungsvorrichtung 2 eine minimale Querschnittsfläche auf, was deren Einführung in die Öffnung 9 besonders einfach macht.

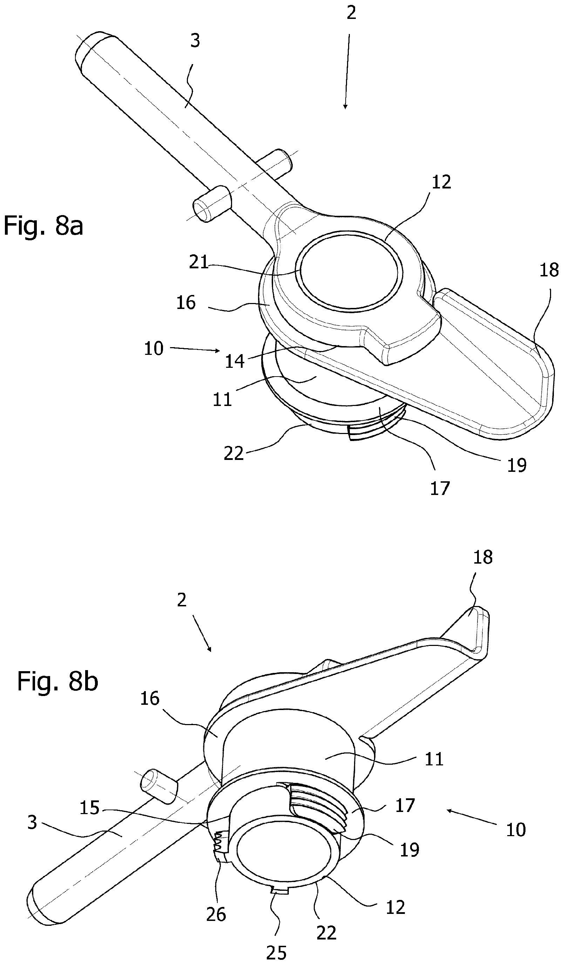

Fig. 8a zeigt eine perspektivische Ansicht der Befestigungsvorrichtung 2 gemäss des zweiten Ausführungsbeispiels mit dem Halteelement 3 in der zweiten Stellung, gemäss welcher das erste Hülsenteilelement 11 relativ zum zweiten Hülsenteilelement 12 um einen Winkel von ungefähr 90 Grad verdreht worden ist. Das Griffelement 18 erstreckt sich gegenüberliegend zum stangenförmigen Körper des Halteelements 3.

Fig. 8b zeigt eine weitere perspektivische Ansicht der Befestigungsvorrichtung 2 mit dem Halteelement 3 in der zweiten Stellung, wobei in dieser Ansicht das zweite Ende 22 des zweiten Hülsenteilelements 12 sowie das zweite Ende 15 des ersten Hülsenteilelements 11 sichtbar sind. In Fig. 8b sind auch der Fortsatz 19 des ersten Hülsenteilelements 11 und der erste Vorsprung 25 und der zweite Vorsprung 26 des zweiten Hülsenteilelements 12 gezeigt. In Fig. 8b ist auch deutlich zu erkennen, dass in der zweiten Stellung die ersten und zweiten Vorsprünge 25, 26 und der Fortsatz 19 ungefähr gegenüberliegend zueinander angeordnet sind. Die Vorsprünge 25, 26 und der Fortsatz 19 sind derart angeordnet, dass sie sie eine Klemmwirkung entfalten, sodass die Befestigungsvorrichtung 2 in der Öffnung 9 fixiert ist. In dieser zweiten Stellung weist somit die Befestigungsvorrichtung 2 eine maximale Querschnittsfläche auf, was einen optimalen Halt in der Öffnung 9 gewährleistet. Zudem ist es möglich, dass der Fortsatz 19 eine Profilierung aufweist, die in die Innenwand der Öffnung 9 eingreift, sodass die Befestigungsvorrichtung in der zweiten Stellung arretiert ist. Auch die ersten und zweiten Vorsprünge können Profilierungen aufweisen, mittels welchen zumindest der Reibungswiderstand an der Innenwand der Öffnung erhöht werden kann.

Insbesondere können die Profilierungen des ersten und zweiten Vorsprungs 25, 26 derart ausgebildet sein, dass sie in die Innenwand der Öffnung 9 eingreifen können. Die Position des ersten Hülsenteilelements 11 relativ zum zweiten Hülsenteilelement 12 in der zweiten Stellung ist abhängig vom Innendurchmesser der Öffnung 9. Die Öffnung 9 kann einen effektiven Durchmesser aufweisen, der vom Nenndurchmesser geringfügig abweicht. Wenn der Nenndurchmesser der Öffnung 9 beispielsweise im Bereich von 10 bis einschliesslich 15 mm liegt, kann der effektive Durchmesser eine Abweichung von bis zu 1 mm in Bezug auf den Nenndurchmesser aufweisen. Die Position des ersten Hülsenteilelements 11 in der zweiten Stellung ist einstellbar, sodass Abweichungen zwischen dem Nenndurchmesser und dem effektiven Durchmesser der Öffnung 9 durch die Befestigungsvorrichtung 2 ausgeglichen werden können. Die Position des Fortsatzes 19 relativ zu den ersten und zweiten Vorsprüngen 25, 26 kann somit in der zweiten Stellung in Abhängigkeit vom effektiven Durchmesser der Öffnung 9 unterschiedlich eingestellt werden.

Fig. 9 zeigt ein Anwendungsbeispiel der Befestigungsvorrichtung gemäss eines der Ausführungsbeispiele. Gemäss des vorliegenden Ausführungsbeispiels wird die Befestigungsvorrichtung für ein Navigationssystem verwendet. Die Befestigungsvorrichtung enthält das Halteelement 3 und einen ersten Tracker 4, der beispielsweise als optischer Marker ausgebildet ist. Eine Therapievorrichtung 6, gemäss des vorliegenden Ausführungsbeispiels ein Pointer 8, ist mit einem zweiten Tracker 5 ausgestattet. Der in Fig. 9 gezeigten zweite Tracker 5 enthält eine Einzelkamera, welche für eine Anwendung der Schattenbildtechnologie ausgebildet ist. Gemäss des vorliegenden Ausführungsbeispiels kann ein erster optischer Marker, der an der Befestigungsvorrichtung 2 angebracht ist, mittels des Navigationssystems erfasst werden. Insbesondere können 6DOF-Informationen betreffend die Position des optischen Markers im Raum und/oder über die relative Position des ersten optischen Markers sowie des zweiten Trackers 5, der einen zweiten optischen Marker enthalten kann, erhältlich sein. Eine Anzeigeeinheit 52 kann verwendet werden, um die Anatomie des Patienten von einem präoperativen Scan auf den am Knochen mittels der Befestigungsvorrichtung 2 befestigten ersten Tracker 4 zu registrieren, bevor eine Therapievorrichtung 6, welche den zweiten Tracker 5 für das Navigationssystem enthält, verwendet wird.

Das Navigationssystem enthält eine Rechnereinheit 51 und eine Anzeigeeinheit 52, um die Position des ersten Trackers 4 auf der Grundlage der Trackingdaten zu berechnen. Die Anzeigeeinheit 52 kann 3D-Modelle der Anatomie und des Trackers 4 anzeigen oder, wenn präoperative Bilder geladen sind, die Position des Trackers 4 in einer oder mehreren Bildschichten 53 anzeigen. Die Rechnereinheit 51 kann in die Anzeigeeinheit 52 integriert oder von dieser getrennt sein. Die Anzeigeeinheit 51 kann beispielsweise als ein Monitor, ein Head-up-Display oder ein Augmented-Reality-Display ausgebildet sein. Das Navigationssystem kann über ein Kabel mit der Rechnereinheit 51 verbunden sein. Alternativ können Trackingdaten drahtlos von einem zweiten Tracker 5, beispielsweise einer Kamera, an die Rechnereinheit 51 übertragen werden.

Für eine Fachperson ist offensichtlich, dass viele weitere Varianten zusätzlich zu den beschriebenen Systemen oder Verfahrensvarianten möglich sind, ohne vom erfinderischen Konzept abzuweichen. Der Gegenstand der Erfindung wird somit durch die vorangehende Beschreibung nicht eingeschränkt und ist durch den Schutzbereich bestimmt, der durch die Ansprüche festgelegt ist. Für die Interpretation der Ansprüche oder der Beschreibung ist die breitest mögliche Lesart der Ansprüche massgeblich. Insbesondere sollen die Begriffe "enthalten" oder "beinhalten" derart interpretiert werden, dass sie sich auf Elemente, Komponenten oder Schritte in einer nicht-ausschliesslichen Bedeutung beziehen, wodurch angedeutet werden soll, dass die Elemente, Komponenten oder Schritte vorhanden sein können oder genutzt werden können, dass sie mit anderen Elementen, Komponenten oder Schritten kombiniert werden können, die nicht explizit erwähnt sind. Wenn die Ansprüche sich auf ein Element oder eine Komponente aus einer Gruppe beziehen, die aus A, B, C bis N Elementen oder Komponenten bestehen kann, soll diese Formulierung derart interpretiert werden, dass nur ein einziges Element dieser Gruppe erforderlich ist, und nicht eine Kombination von A und N, B und N oder irgendeiner anderen Kombination von zwei oder mehr Elementen oder Komponenten dieser Gruppe.

## Patentansprüche

1. Befestigungsvorrichtung (2) zur Befestigung einer Therapievorrichtung (6) in einer in einem Knochen angeordneten Öffnung (9) enthaltend ein Hülsenelement (10), welches zur Aufnahme in der Öffnung (9) ausgebildet ist, wobei das Hülsenelement (10) ein erstes Hülsenteilelement (11) und ein zweites Hülsenteilelement (12) enthält, wobei das erste Hülsenteilelement (11) im Einbauzustand das zweite Hülsenteilelement (12) umgibt, wobei das erste Hülsenteilelement (11) als ein zylinderförmiger Hohlkörper mit einer Längsachse ausgebildet ist, wobei das erste Hülsenteilelement (11) ein erstes Ende (14) und ein zweites Ende (15) aufweist, wobei das erste Ende (14) des ersten Hülsenteilelements (11) ein Griffelement (18) umfasst, wobei das zweite Ende (15) des ersten Hülsenteilelements (11) einen Fortsatz (19) umfasst, wobei das zweite Hülsenteilelement (12) als ein zylinderförmiger Hohlkörper mit einer Längsachse ausgebildet ist, wobei das zweite Hülsenteilelement (12) eine Vorsprunganordnung (25, 26) enthält, wobei das erste Hülsenteilelement (11) und das zweite Hülsenteilelement (12) derart um einander drehbar sind, dass die Vorsprunganordnung (25, 26) in einer ersten Stellung angrenzend an den Fortsatz (19) angeordnet ist und die Vorsprunganordnung (25, 26) in einer zweiten Stellung einen Abstand zum Fortsatz (19) aufweist.

2. Befestigungsvorrichtung nach Anspruch 1, wobei das erste Hülsenteilelement (11) einen ersten Flansch (16) sowie einen zweiten Flansch (17) enthält.

3. Befestigungsvorrichtung nach Anspruch 2, wobei der Fortsatz (19) den zweiten Flansch (17) in Richtung der Längsachse des zylinderförmigen Hohlkörpers des ersten Hülsenteilelements (11) überragt.

4. Befestigungsvorrichtung nach einem der vorhergehenden Ansprüche, wobei der Fortsatz (19) sich über einen Teil eines Umfangs des ersten Hülsenteilelements (11) erstreckt.

5. Befestigungsvorrichtung einem der vorhergehenden Ansprüche, wobei der Fortsatz (19) eine Segmentlänge aufweist, die im Bereich von 10% bis einschliesslich 40 % des Umfangs des zylinderförmigen Hohlkörpers liegt.

6. Befestigungsvorrichtung einem der vorhergehenden Ansprüche, wobei der Fortsatz (19) eine axiale Länge aufweist, die im Bereich von 5% bis einschliesslich 50% des zwischen dem ersten Ende (14) und dem zweiten Ende (15) in Richtung einer Längsachse des ersten Hülsenteilelements (11) gemessenen Abstandes liegt.

7. Befestigungsvorrichtung einem der vorhergehenden Ansprüche, wobei der Fortsatz (19) eine Profilierung (20) auf der Aussenseite aufweist.

8. Befestigungsvorrichtung einem der vorhergehenden Ansprüche, wobei das zweite Hülsenteilelement (12) eine Aussenseite aufweist, die zumindest einen Absatz (24) enthält.

9. Befestigungsvorrichtung einem der vorhergehenden Ansprüche, wobei das zweite Hülsenteilelement (12) ein Halteelement (3) enthält.

10. Befestigungsvorrichtung einem der vorhergehenden Ansprüche, wobei die Vorsprunganordnung einen ersten Vorsprung (25) und einen zweiten Vorsprung (26) umfasst, wobei die Vorsprunganordnung (25, 26) eine Profilierung enthalten kann.

11. Befestigungsvorrichtung einem der vorhergehenden Ansprüche, wobei das zweite Hülsenteilelement (12) einen Innendurchmesser aufweist, der mindestens 3 mm beträgt.

12. Befestigungsvorrichtung einem der vorhergehenden Ansprüche, wobei das erste Hülsenteilelement (11) einen Längsschlitz (13) aufweist, wobei sich der Längsschlitz (13) vom ersten Ende (14) des ersten Hülsenteilelements (11) zum zweiten Ende (15) des ersten Hülsenteilelements (11) erstreckt.

13. Befestigungsvorrichtung einem der vorhergehenden Ansprüche, wobei das zweite Hülsenteilelement (12) einen Längsschlitz (23) aufweist, wobei sich der Längsschlitz (23) von einem ersten Ende (21) des zweiten Hülsenteilelements (12) zu einem zweiten Ende (22) des zweiten Hülsenteilelements (12) erstreckt.

14. Navigationssystem, enthaltend eine Befestigungsvorrichtung (2) nach einem der vorhergehenden Ansprüche.

15. Navigationssystem nach Anspruch 14, wobei die Befestigungsvorrichtung (2), zur Befestigung eines ersten Trackers (4) in einer in einem Knochen angeordneten Öffnung (9) ausgebildet ist, wobei das Navigationssystem eine Therapievorrichtung (6), welche einen zweiten Tracker (5) enthält, ein Trackingsystem, eine Rechnereinheit (51) und eine Anzeigeeinheit (52) umfasst, wobei mittels des Trackingsystems die Position der Therapievorrichtung (6) relativ zu der Befestigungsvorrichtung (2) erfassbar ist, wobei die Rechnereinheit (51) zur Berechnung der Position der Therapievorrichtung (6) in einem anatomischen Koordinatenrahmen ausgebildet ist, wobei die Anzeigeeinheit (52) ausgebildet ist, eine virtuelle Position der Therapievorrichtung (6) in Bezug auf registrierte präoperative Bilder oder 3D-Modelle anzuzeigen.
